## Europäisches Patentamt

## European Patent Office

## Office européen des brevets

(19)

(11) Veröffentlichungsnummer : **0 131 991**
B1

(12) **EUROPÄISCHE PATENTSCHRIFT**

(45) Veröffentlichungstag der Patentschrift :
03.12.86

(51) Int. Cl.⁴ : **C 07 C 67/03**, C 07 C 69/24,
C 07 C 69/52

(21) Anmeldenummer : 84200937.5

(22) Anmeldetag : 29.06.84

(54) Kontinuierliches Alkoholyseverfahren.

(30) Priorität : 12.07.83 DE 3325066
12.01.84 DE 3400766
26.04.84 DE 3415529

(43) Veröffentlichungstag der Anmeldung :
23.01.85 Patentblatt 85/04

(45) Bekanntmachung des Hinweises auf die Patenterteilung : 03.12.86 Patentblatt 86/49

(84) Benannte Vertragsstaaten :
AT BE CH DE FR GB IT LI LU NL SE

(56) Entgegenhaltungen :
DE-A- 3 107 318

(73) Patentinhaber : METALLGESELLSCHAFT AG
Reuterweg 14 Postfach 3724
D-6000 Frankfurt/M.1 (DE)

(72) Erfinder : Schmidt, Hans-Joachim, Dr.
Im Trutz 36
D-6000 Frankfurt am Main (DE)
Erfinder : Fricke, Wolfram
Rüsselsheimer Strasse 47
D-6097 Treburg (DE)

(74) Vertreter : Rieger, Harald, Dr.
Reuterweg 14
D-6000 Frankfurt am Main (DE)

## Beschreibung

Die Erfindung betrifft ein kontinuierliches katalytisches Verfahren zur Alkoholyse von Fettsäureglyceriden mit einwertigen Alkoholen, die ein bis vier C-Atome enthalten, bei erhöhter Temperatur.

Fettsäureglyceride sind Ester von Fettsäuren mit Glycerin wie sie z. B. als Triglyceride in natürlichen pflanzlichen oder tierischen Fetten und Ölen vorkommen. Derartige Fettsäureglyceride werden industriell mit einwertigen Alkoholen, die ein bis vier C-Atome enthalten können, hauptsächlich jedoch mit Methanol umgesetzt und ergeben die Methyl- bis Butylester der entsprechenden Fettsäuren. Gleichzeitig entsteht dabei Glycerin als wertvolles Nebenprodukt. Die Fettsäureester sind, soweit sie nicht als solche Verwendung finden, Zwischenprodukte bei der Herstellung von Fettalkoholen, Fettsäureamiden, epoxidierten Estern u. a.

Bisher werden Fettsäureester von einwertigen Alkoholen, die ein bis vier C-Atome enthalten, durch Alkoholyse von Fettsäureglyceriden kontinuierlich hergestellt, indem man die Fettsäureglyceride und den entsprechenden Alkohol im Gleichstrom bei erhöhter Temperatur und ggfs. bei erhöhtem Druck durch einen Reaktor fördert. Die Alkoholysereaktion wird durch Katalysatoren beschleunigt. Man verwendet dazu Alkalien, wie z. B. Natriumhydroxid oder Natriummethylat, es werden aber auch Magnesium-, Calcium-, Zink- und Zinnverbindungen eingesetzt, ebenso saure Katalysatoren wie Salzsäure, Bortrifluorid u. a. (Ullmann, Enzyklopädie der Technischen Chemie, 4. Aufl. ; Henkel, Fettalkohole, 1981 ; Vegetable Oil Fuels, Proceedings of the International Conference on Plant and Vegetable Oils as Fuels, Fargo, 1982). Bei der Durchführung der Alkoholysereaktion im Gleichstrom ist es notwendig, einen großen Überschuß an einwertigen Alkoholen anzuwenden, um einen hohen Umsatz zu den Fettsäureestern zu erhalten. Nach der Reaktion befindet sich der Alkoholüberschuß in dem aus zwei flüssigen Phasen bestehenden Reaktionsgemisch, der leichten esterhaltigen Phase und der schweren glycerinhaltigen Phase, aus denen der Alkoholüberschuß energieaufwendig durch Verdampfen zurückgewonnen werden muß, um zur erneuten Umsetzung mit den Fettsäureglyceriden eingesetzt werden zu können.

Der Erfindung liegt die Aufgabe zugrunde, den Alkoholüberschuß bei der Alkoholyse von Fettsäureglyceriden zu senken und gleichzeitig eine hohe Ausbeute an Fettsäureestern zu erhalten.

Diese Aufgabe wird erfindungsgemäß dadurch gelöst, daß die einwertigen Alkohole mit einem Teil der bei der Reaktion entstehenden glycerinhaltigen schweren Phase gemischt und im Gegenstrom zu den Fettsäureglyceriden bzw. der daraus entstehenden esterhaltigen leichten Phase geführt werden.

Eine weitere Senkung des Alkoholüberschusses wird dadurch erreicht, daß man die rückgeführte glycerinhaltige schwere Phase vor der Mischung mit den einwertigen Alkoholen zur Extraktion der noch in der leichten Phase gelösten einwertigen Alkohole benutzt.

Eine weitere vorteilhafte Ausgestaltung des erfindungsgemäßen Verfahrens sieht vor, die leichte esterhaltige Phase nach der Reaktion auf eine Temperatur unterhalb der Reaktionstemperatur zu kühlen, das dabei anfallende Glycerin-Alkohol-Gemisch von der leichten esterhaltigen Phase abzutrennen und zusammen mit der rückgeführten glycerinhaltigen schweren Phase und den einwertigen Alkoholen in den Reaktor zu fördern.

Für die Durchführung des erfindungsgemäßen Verfahrens ist es weiterhin von Vorteil, das Gemisch aus den einwertigen Alkoholen und der rückgeführten glycerinhaltigen schweren Phase in einen im Kreislauf geführten Strom der esterhaltigen leichten Phase einzuspeisen und mit diesen dem Reaktor zurückzuführen.

Außerdem ist es zweckmäßig, die einwertigen Alkohole mit der den Gegenstrom verlassenden esterhaltigen leichten Phase zu mischen und zur Reaktion zu bringen, bevor sie mit der bei der Reaktion entstehenden glycerinhaltigen schweren Phase gemischt und dem Gegenstromreaktor zugeführt werden.

Ein weiterer Vorteil kann erreicht werden, wenn die Fettsäureglyceride zunächst mit der bei der Reaktion entstehenden glycerinhaltigen schweren Phase gemischt, von schädlichen Begleitstoffen befreit, danach von der schweren Phase wieder abgetrennt und dann erst in den Gegenstromreaktor eingeführt werden.

Schließlich ist es bei dem erfindungsgemäßen Verfahren nach Anspruch 1 möglich, gleichzeitig mehr als eine Maßnahme der Ansprüche 2 bis 6 kombiniert anzuwenden.

Die Durchführung des erfindungsgemäßen Verfahrens geschieht zweckmäßigerweise in einer Kolonne, die im einfachsten Fall aus einem senkrecht stehenden leeren Rohr besteht. Die Reaktion kann aber auch in einem mehrstufigen Mixer-Settler durchgeführt werden, wie es bei der flüssig-flüssig Gegenstrom-Extraktion üblich ist.

Die mit der Erfindung erzielten Vorteile bestehen insbesondere darin, daß für die Rückgewinnung der überschüssigen einwertigen Alkohole aus den Endprodukten weniger Energie und damit weniger Betriebskosten aufgebracht werden müssen. Durch Erhöhung der Menge an rückgeführter glycerinhaltiger schwerer Phase wird die Menge an gelöstem überschüssigem Alkohol und gelöstem Glycerin in der esterhaltigen leichten Phase gesenkt, was die Aufarbeitung zu den Fettsäureestern erleichtert und ebenfalls zur Kostensenkung beiträgt. Da sich der Katalysator, vor allem bei der Verwendung von Natriumhydroxid oder Natriummethylat, nach der Reaktion hauptsächlich gelöst in der glycerinhaltigen schweren Phase befindet, wird durch die Rückführung der schweren Phase die Katalysatorkonzentration im Reaktor erhöht, was zu einer Beschleunigung der Alkoholysereaktion führt oder zu einer Senkung des Katalysatorverbrauchs ausgenutzt werden kann.

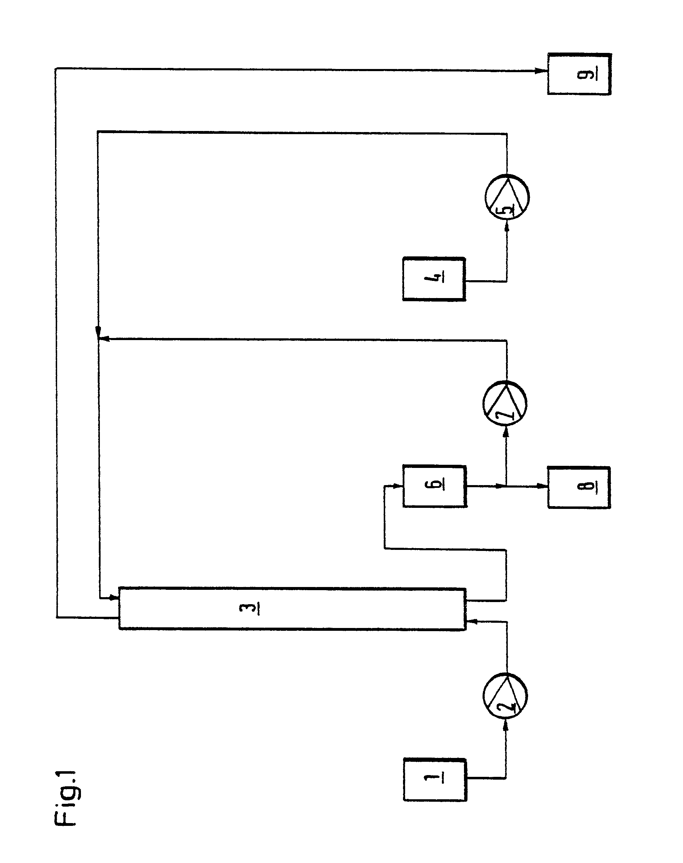

Das erfindungsgemäße Verfahren wird anhand der Figuren 1 bis 7 näher erläutert.

Die Ziffern in den Figuren haben folgende Bedeutung :

1. Vorratsbehälter für Fettsäureglyceride
2. Dosierpumpe für Fettsäureglyceride
3. Alkoholysereaktor
4. Vorratsbehälter für einwertige Alkohole mit Katalysator
5. Dosierpumpe für einwertige Alkohole
6. Zwischenbehälter für glycerinhaltige schwere Phase
7. Dosierpumpe für glycerinhaltige schwere Phase
8. Auffangbehälter für glycerinhaltige schwere Phase
9. Auffangbehälter für esterhaltige leichte Phase
10. Mischer für glycerinhaltige schwere und esterhaltige leichte Phase
11. Trennapparat für leichte und schwere Phase
12. Kühler für esterhaltige leichte Phase
13. Trennapparat für leichte und schwere Phase
14. Kreislaufpumpe für esterhaltige leichte Phase
15. Reaktor
16. Mischapparat
17. Pumpe
18. Trennapparat
19. Auffangbehälter.

In Figur 1 ist das erfindungsgemäße Verfahren gemäß Anspruch 1 dargestellt. Die Fettsäureglyceride werden aus dem Vorratsbehälter 1 mit der Dosierpumpe 2 in den Alkoholysereaktor 3 gefördert. Die hauptsächlich aus Fettsäureester bestehende leichte Phase gelangt aus dem Reaktor 3 in den Auffangbehälter 9. Im Gegenstrom zur esterhaltigen leichten Phase wird im Reaktor 3 ein Gemisch aus einwertigem Alkohol und rückgeführter glycerinhaltiger schwerer Phase geführt. Die katalysatorhaltigen einwertigen Alkohole werden aus dem Vorratsbehälter 4 mit der Dosierpumpe 5 und die glycerinhaltige schwere Phase aus dem Zwischenbehälter 6 mit der Dosierpumpe 7 zum Reaktor 3 gepumpt. Produzierte überschüssige glycerinhaltige schwere Phase wird im Behälter 8 gesammelt.

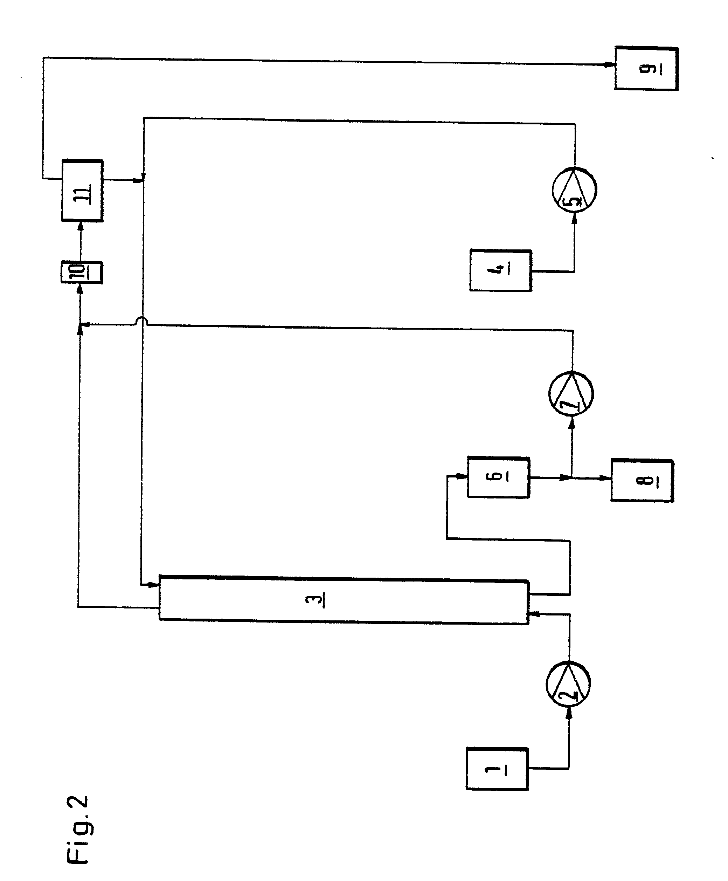

Figur 2 zeigt eine vorteilhafte Ausgestaltung des erfindungsgemäßen Verfahrens. Die den Reaktor verlassende esterhaltige leichte Phase wird zur Extraktion von noch gelöstem Alkohol im Mischer 10 mit rückgeführter glycerinhaltiger schwerer Phase gemischt. Im Trennapparat 11 erfolgt die Trennung von leichter und schwerer Phase. Die esterhaltige leichte Phase gelangt in den Auffangbehälter 9, während die schwere Phase mit dem extrahierten Alkohol zusammen mit dem einwertigen Alkohol dem Reaktor 3 zugeführt wird.

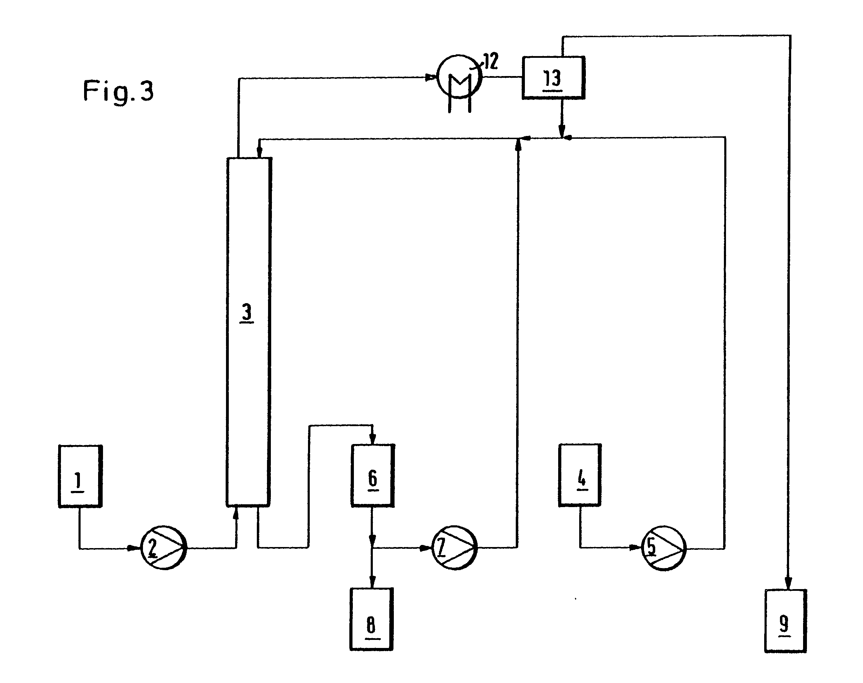

Die weitere Ausgestaltung des erfindungsgemäßen Verfahrens in Figur 3 zeigt den Kühler 12, in dem die den Reaktor verlassende esterhaltige leichte Phase gekühlt wird. Das dabei ausfallende Gemisch von Glycerin und Alkohol wird im Trennapparat 13 als schwere Phase abgetrennt und zusammen mit der rückgeführten glycerinhaltigen schweren Phase und den einwertigen Alkoholen zum Reaktor 3 gefördert. Die esterhaltige leichte Phase gelangt wiederum in den Auffangbehälter 9.

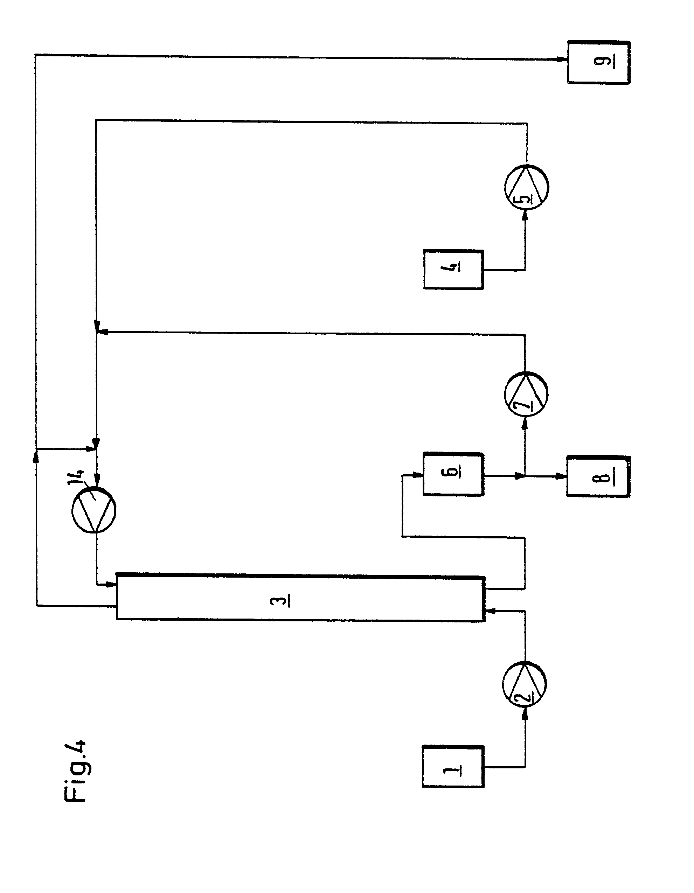

Figur 4 zeigt in weiterer Ausgestaltung der Erfindung eine Kreislaufpumpe 14, mit der ein Kreislaufstrom von esterhaltiger leichter Phase aufrechterhalten wird. In diesem Kreislauf wird das Gemisch aus einwertigen Alkoholen und rückgeführter glycerinhaltiger schwerer Phase eingespeist.

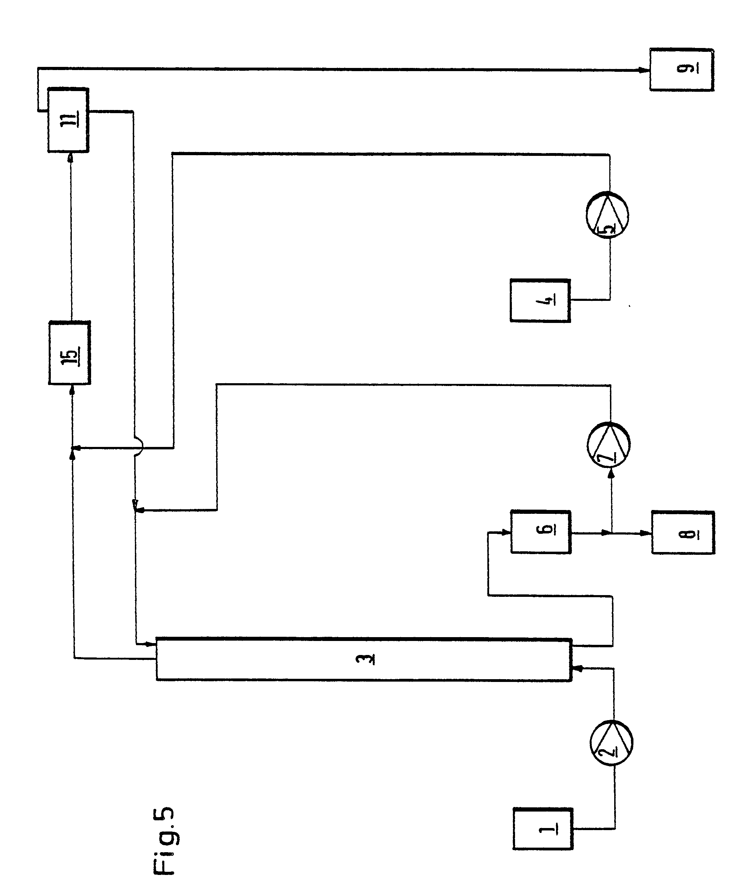

In Figur 5 ist eine alternative Ausgestaltung des erfindungsgemäßen Verfahrens dargestellt. Dabei werden wie bisher Fettsäureglyceride aus dem Vorratsbehälter 1 mit der Dosierpumpe 2 in den Gegenstromreaktor gefördert. Die hauptsächlich aus Fettsäureester bestehende leichte Phase wird oben aus dem Gegenstromreaktor abgezogen und in Abänderung der zuvor beschriebenen Ausführungsformen zusammen mit einem dem Vorratsbehälter 4 mittels Dosierpumpe 5 entnommenen Strom an einwertigen Alkoholen mit eingemischtem Katalysator dem Reaktor 15 zugeführt, wo die fast ausreagierte esterhaltige leichte Phase mit den frischen Alkoholen vollständig ausreagiert. Im Trennapparat 11 wird sodann die leichte Phase abgezogen und dem Auffangbehälter 9 zugeführt. Die Alkohole mit den aus der Endreaktion stammenden Anteilen an glycerinhaltiger schwerer Phase, werden mit der aus dem Zwischenbehälter 6 mittels Pumpe 7 abgezogenen glycerinhaltigen schweren Phase gemischt und zusammen in den Gegenstromreaktor 3 eingeführt. Mit 8 ist der Auffangbehälter für die glycerinhaltige schwere Phase bezeichnet.

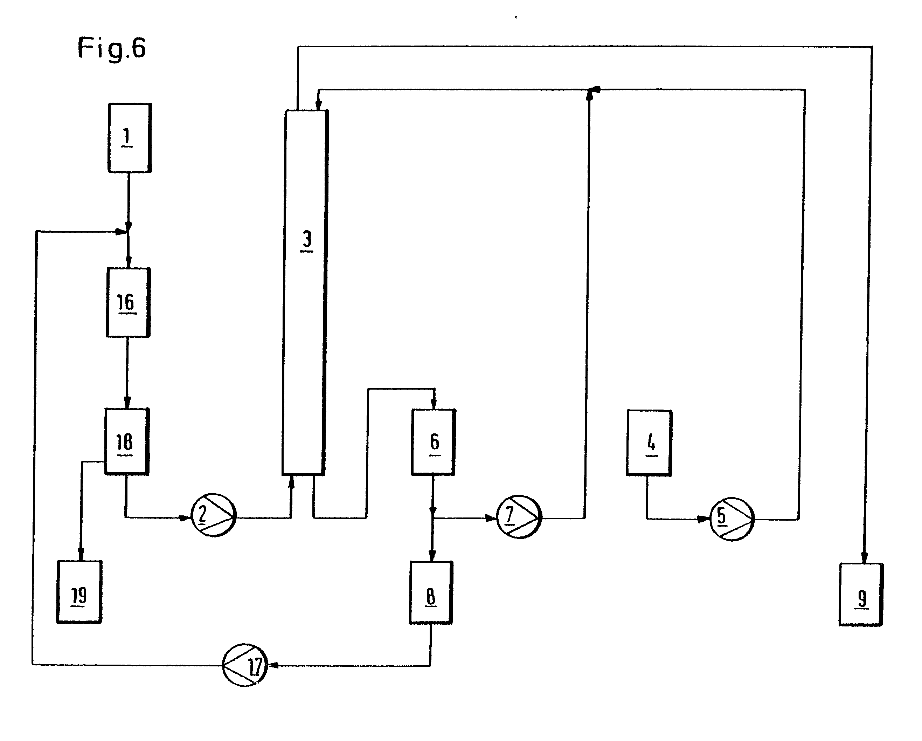

In Figur 6 schließlich ist noch eine weitere, unabhängig von den bisher beschriebenen Maßnahmen anwendbare Alternative dargestellt. Dabei werden die Fettsäureglyceride aus dem Vorratsbehälter 1 zunächst in einen Mischapparat 16 gefördert, in den mittels Pumpe 17 auch glycerinhaltige schwere Phase aus dem Auffangbehälter 8 eingebracht wird. Im Mischapparat 16 werden Begleitstoffe der Fettsäureglyceride, die für die Reaktion schädlich sind, in die glycerinhaltige schwere Phase überführt. Anschließend wird im Trennapparat 18 die glycerinhaltige schwere Phase zusammen mit den schädlichen Begleitstoffen wieder abgetrennt und die insoweit gereinigten Fettsäureglyceride mittels Dosierpumpe 2 in den Gegenstromreaktor 3 gefördert. Die hauptsächlich aus Fettsäureester bestehende leichte Phase gelangt aus dem Reaktor 3 in den Auffangbehälter 9. Im Gegenstrom zur esterhaltigen leichten Phase wird im Reaktor 3 ein Gemisch aus einwertigem Alkohol und rückgeführter glycerinhaltiger schwerer Phase geführt. Die katalysatorhaltigen einwertigen Alkohole werden aus dem Vorratsbehälter 4 mit der Dosierpumpe 5 und die glycerinhaltige schwere Phase aus dem Zwischenbehälter 6 mit der Dosierpumpe 7 zum Reaktor 3 gepumpt. Produzierte überschüssige glycerinhaltige schwere Phase wird im Behälter 8 gesammelt. Mit 19 ist ein weiterer Auffangbehälter für die glycerinhaltige schwere Phase bezeichnet.

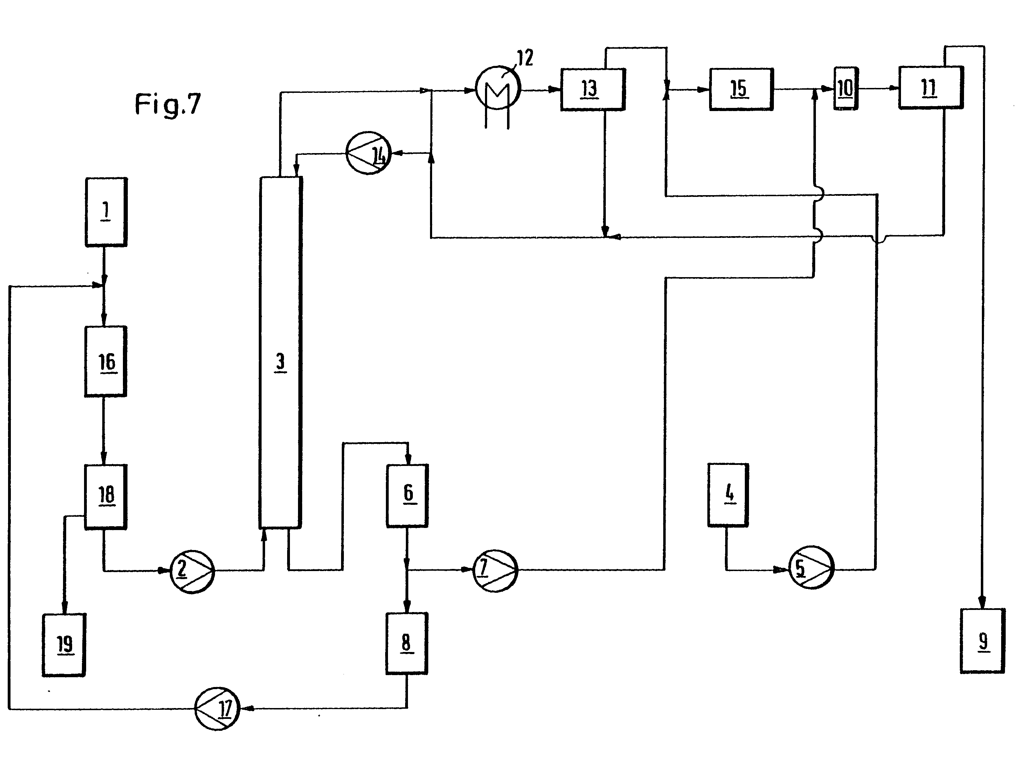

In Figur 7 ist schließlich eine Kombination aller bisher beschriebenen Verfahrensschritte dargestellt. Die Fettsäureglyceride werden aus dem Vorratsbehälter 1 in den Mischapparat 16 gefördert, in den mittels Pumpe 17 auch glycerinhaltige schwere Phase aus dem Auffangbehälter 8 eingebracht wird. Nach Überführung der schädlichen Begleitelemente aus den Fettsäureglyceriden in die glycerinhaltige schwere Phase, werden diese beiden Stoffe im Trennapparat 18 wieder getrennt, die insoweit gereinigten Fettsäureglyceride mittels Dosierpumpe 2 in den Gegenstromreaktor gefördert und die glycerinhaltige schwere Phase im Auffangbehälter 19 gesammelt. Die einwertigen Alkohole befinden sich zusammen mit dem Katalysator in dem Vorratsbehälter 4 und werden mittels Pumpe 5 in den Prozeß eingeschleust. Die glycerinhaltige schwere Phase gelangt über den Zwischenbehälter 6 zum Teil in den Auffangbehälter 8 oder wird mittels Pumpe 7 in den Prozeß zurückgeführt. Die esterhaltige leichte Phase wird schließlich im Behälter 9 aufgefangen.

Eine erste Maßnahme zur Verbesserung des Grundprozesses besteht darin, die am Kopf des Gegenstromreaktors abgezogene esterhaltige leichte Phase mittels Pumpe 14 im Kreislauf zu führen. Eine zweite Maßnahme besteht darin, die esterhaltige leichte Phase nach Austritt aus dem Gegenstromreaktor bzw. dem über die Pumpe 14 laufenden Kreislauf im Kühler 12 auf eine Temperatur unterhalb der Reaktionstemperatur abzukühlen und dann in Trennapparat 13 die leichte esterhaltige Phase von dem infolge Temperaturabsenkung ausfallenden Glycerin-Alkohol-Gemisch abzutrennen. Während das Glycerin-Alkohol-Gemisch in den Gegenstromreaktor 3 zurückgeführt wird, wird die leichte esterhaltige Phase mit den frischen Alkoholen gemischt und im Reaktor 15 vollständig ausreagiert. Die fertig ausreagierte leichte Phase wird zusammen mit den darin enthaltenden Rückständen mit dem über die Pumpe 7 zugeführten Strom der glycerinhaltigen schweren Phase im Mischer 10 gemischt, worauf im Trennapparat 11 die leichte Phase abgetrennt und in den Behälter 9 geführt wird, während die schwere glycerinhaltige Phase zusammen mit den Alkoholen in den Gegenstromreaktor weitergeleitet wird, in den sie zusammen mit dem aus dem Trennapparat 13 ankommenden Strom an schwerer glycerinhaltiger Phase sowie mit der im Kreislauf geführten (Pumpe 14) leichten esterhaltigen Phase eintritt.

Der in den Figuren 1 bis 7 schematisch dargestellte Alkoholysereaktor 3, in dem die einwertigen Alkohole mit einem Teil der bei der Reaktion entstehenden glycerinhaltigen schweren Phase im Gegenstrom zu den Fettsäureglyceriden bzw. der daraus entstehenden esterhaltigen leichten Phase geführt werden, besteht im einfachsten Fall aus einer Kolonne, d. h. aus einem senkrecht stehenden Rohr mit oder ohne Einbauten bzw. Füllkörpern. In diesem Fall werden die Fettsäureglyceride unten in den Reaktor eingespeist und die daraus entstehende esterhaltige leichte Phase wird oben am Reaktor abgezogen, während das Gemisch aus einwertigen Alkoholen und rückgeführter glycerinhaltiger schwerer Phase oben dem Reaktor zugeführt und die entstehende glycerinhaltige schwere Phase unten abgeführt wird. Die Gegenstromführung wird durch einen Trennschichtregler, der die Trennschicht zweckmäßigerweise im unteren Kolonnenteil hält, sichergestellt. Der Stoffaustausch in der Kolonne kann durch den Einsatz einer pulsierten Kolonne oder durch einen Rotating-Disc-Contactor gefördert werden.

Weiterhin kann das erfindungsgemäße Verfahren in einem Mixer-Settler durchgeführt werden, wie er bei der flüssig-flüssig Gegenstromextraktion eingesetzt wird und bei dem die Gegenstromführung der beiden Phasen durch Zwangsförderung erfolgt.

## Patentansprüche

1. Verfahren zur kontinuierlichen katalytischen Alkoholyse von Fettsäureglyceriden mit einwertigen Alkoholen, die 1 bis 4 C-Atome enthalten, bei erhöhter Temperatur, dadurch gekennzeichnet, daß man die Fettsäureglyceride von unten in einen Reaktor leitet, aus dem unteren Bereich des Reaktors eine glycerinhaltige schwere Phase abzieht, einen Teil davon mit dem einwertigen Alkohol und einem Katalysator zu einem Gemisch vereinigt und das Gemisch dem oberen Bereich des Reaktors aufgibt, daß man den einwertigen Alkohol und die Fettsäureglyceride sowie eine daraus entstehende esterhaltige leichte Phase im Reaktor im Gegenstrom führt und im oberen Bereich des Reaktors die esterhaltige leichte Phase abzieht.

2. Verfahren nach Anspruch 1, dadurch gekennzeichnet, daß die rückgeführte glycerinhaltige schwere Phase vor der Mischung mit den einwertigen Alkoholen zur Extraktion der noch in der leichten Phase gelösten einwertigen Alkohole benutzt wird.

3. Verfahren nach Anspruch 1, dadurch gekennzeichnet, daß die leichte esterhaltige Phase nach der Reaktion auf eine Temperatur unterhalb der Reaktionstemperatur gekühlt, das dabei anfallende Glycerin-Alkohol-Gemisch von der leichten esterhaltigen Phase abgetrennt und zusammen mit der rückgeführten glycerinhaltigen schweren Phase und den einwertigen Alkoholen in den Reaktor gefördert wird.

4. Verfahren nach Anspruch 1, dadurch gekennzeichnet, daß das Gemisch aus den einwertigen Alkoholen und der rückgeführten glycerinhaltigen schweren Phase in einen im Kreislauf

gefÜhrten Strom von esterhaltiger leichter Phase eingespeist und mit diesem in den Reaktor zurückgeführt wird.

5. Verfahren nach Anspruch 1, dadurch gekennzeichnet, daß die einwertigen Alkohole mit der den Gegenstromreaktor verlassenden esterhaltigen leichten Phase gemischt und zur Reaktion gebracht werden, bevor sie mit der bei der Reaktion entstehenden Phase gemischt und dem Gegenstromreaktor zugeführt werden.

6. Verfahren nach Anspruch 1, dadurch gekennzeichnet, daß die Fettsäureglyceride zunächst mit der bei der Reaktion entstehenden glycerinhaltigen schweren Phase gemischt, von schädlichen Begleitstoffen befreit, danach von der schweren Phase wieder abgetrennt und dann erst in den Gegenstromreaktor eingeführt werden.

7. Verfahren nach Anspruch 1, dadurch gekennzeichnet, daß es gleichzeitig die Merkmale von mindestens zwei der Ansprüche 2 bis 6 aufweist.

8. Verfahren nach einem der Ansprüche 1 bis 7, dadurch gekennzeichnet, daß die Reaktion in einem aus einer Kolonne bestehenden Reaktor durchgeführt wird.

9. Verfahren nach einem der Ansprüche 1 bis 7, dadurch gekennzeichnet, daß die Reaktion in einem mehrstufigen Mixer-Settler durchgeführt wird.

## Claims

1. A process of continuously alcoholizing fatty acid glycerides with monohydric alcohols containing one to four carbon atoms at elevated temperature, characterized in that said fatty acid glycerides are fed from below into a reactor, from the lower portion of the reactor a glycerine-containing heavy phase is withdrawn and a portion of said heavy phase is mixed with the monohydric alcohol and a catalyst, the mixture is fed into the upper portion of the reactor, the monohydric alcohol and the fatty acid glycerides and an ester-containing light phase formed therewith are conducted in countercurrent contact in the reactor, and from the upper portion of the reactor the ester-containing light phase is withdrawn.

2. A process according to claim 1, characterized in that the glycerine-containing heavy phase to be recycled is used to extract the monohydric alcohols which are still dissolved in the light phase before said heavy phase is mixed with the monohydric alcohols.

3. A process according to claim 1, characterized in that the ester-containing light phase formed by the reaction is cooled to a temperature below the reaction temperature so that a glycerine-alcohol-mixture segregates, that mixture is separated from the ester-containing light phase and together with the glycerine-containing heavy phase to be recycled and the monohydric alcohols is supplied to the reactor.

4. A process according to claim 1, characterized in that the mixture of the monohydric alcohols and the glycerine-containing heavy phase to be recycled is fed to a circulated stream of the ester-containing light phase and is supplied to the reactor in said stream.

5. A process according to claim 1, characterized in that the monohydric alcohols are mixed and reacted with the ester-containing light phase which is withdrawn from the countercurrent reactor before said monohydric alcohols are mixed with the glycerine-containing heavy phase produced by the reaction and supplied to the countercurrent reactor.

6. A process according to claim 1, characterized in that the fatty acid glycerides are first mixed with the glycerine-containing heavy phase produced by the reaction and deleterious impurities are removed from said fatty acid glycerides, which are subsequently separated from the heavy phase and then are supplied to the countercurrent reactor.

7. A process according to claim 1, characterized by the combined features of at least two of the claims 2 to 6.

8. A process according to any of claims 1 to 7, characterized in that the reaction is carried out in a reactor which consists of a column.

9. A process according to any of claims 1 to 7, characterized in that the reaction is carried out in a multistage mixer-settler.

## Revendications

1. Procédé d'alcoolyse catalytique en continu de glycérides d'acides gras, par des monoalcools qui ont de 1 à 4 atomes de carbone, en procédant à température élevée, caractérisé en ce que l'on envoie les glycérides d'acides gras par le bas dans un réacteur, on soutire de la partie inférieure du réacteur une phase lourde contenant de la glycérine, on en réunit une partie au monoalcool et à un catalyseur pour former un mélange et on charge le mélange à la partie supérieure du réacteur, on envoie à contre-courant dans le réacteur le monoalcool et les glycérides d'acides gras, ainsi qu'une phase légère en provenant et contenant un ester, et on soutire dans la partie supérieure du réacteur la phase légère, contenant un ester.

2. Procédé suivant la revendication 1, caractérisé en ce qu'il consiste à utiliser la phase lourde recyclée contenant de la glycérine, avant le mélange aux monoalcools, pour extraire les monoalcools encore dissous dans la phase légère.

3. Procédé suivant la revendication 1, caractérisé en ce qu'il consiste à refroidir la phase légère, contenant un ester après la réaction, jusqu'à une température inférieure à la température réactionnelle, à séparer le mélange de glycérine et d'alcool qui se forme de la phase légère, contenant un ester et à l'envoyer au réacteur ensemble avec la phase lourde recyclée, contenant de la glycérine et avec les monoalcools.

4. Procédé suivant la revendication 1, caractérisé en ce qu'il consiste à introduire le mélange constitué des monoalcools et de la phase lourde recyclée, contenant de la glycérine, dans un courant circulant en circuit fermé de la phase légère, contenant un ester et à le recycler dans le réacteur avec ce courant.

5. Procédé suivant la revendication 1, caractérisé en ce qu'il consiste à mélanger les monoalcools à la phase légère, contenant un ester et quittant le réacteur à contre-courant, et à les mettre à réagir sur cette phase, avant qu'ils soient mélangés à la phase se formant lors de la réaction et envoyés en réacteur à contre-courant.

6. Procédé suivant la revendication 1, caractérisé en ce qu'il consiste à mélanger d'abord les glycérides d'acides gras à la phase lourde, contenant de la glycérine et formée lors de la réaction, à les débarrasser d'impuretés nuisibles, puis à les reséparer de la phase lourde, et ensuite seulement à les envoyer au réacteur à contre-courant.

7. Procédé suivant la revendication 1, caractérisé en ce qu'il présente simultanément les caractéristiques d'au moins deux des revendications 2 à 6.

8. Procédé suivant l'une des revendications 1 à 7, caractérisé en ce qu'il consiste à effectuer la réaction dans un réacteur constitué d'une colonne.

9. Procédé suivant l'une des revendications 1 à 7, caractérisé en ce qu'il consiste à effectuer la réaction dans un mélangeur-décanteur à étages multiples.

Fig.1

Fig.2

Fig. 3

Fig.4

Fig.5

0 131 991

Fig.6

0 131 991

Fig.7

0 131 991